(19) **Europäisches Patentamt**
**European Patent Office**
**Office européen des brevets**

(11) **EP 2 162 093 B1**

(12) **EUROPEAN PATENT SPECIFICATION**

(45) Date of publication and mention
of the grant of the patent:
**21.08.2013 Bulletin 2013/34**

(51) Int Cl.:
***A61F 2/16*** *(2006.01)*

(21) Application number: **08769875.9**

(22) Date of filing: **30.05.2008**

(86) International application number:
**PCT/US2008/065269**

(87) International publication number:
**WO 2008/150982 (11.12.2008 Gazette 2008/50)**

(54) **Diffractive intraocular lens and method**

Diffraktive Intraokularlinse und Verfahren

Lentille intra-oculaire diffractive et procédé

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **01.06.2007 US 756973**

(43) Date of publication of application:
**17.03.2010 Bulletin 2010/11**

(73) Proprietor: **Bausch & Lomb Incorporated
Rochester, NY 14604-2701 (US)**

(72) Inventor: **ALTMANN, Griffith, E.
Pittsford, NY 14534 (US)**

(74) Representative: **Vossius & Partner
Siebertstrasse 4
81675 München (DE)**

(56) References cited:
**EP-A- 0 343 067      US-A1- 2004 252 274
US-A1- 2006 098 163**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**EP 2 162 093 B1**

**Description**

Field of Invention

The present invention relates to diffractive intraocular lenses (IOLs), and more particularly to diffractive multifocal IOLs.

Background of the Invention

IOLs are artificial lenses used to replace natural lenses of patients when their natural lenses are diseased or otherwise impaired. Under some circumstances a natural lens may remain in a patient's eye together with an implanted IOL. IOLs may be placed in either the posterior chamber or the anterior chamber of an eye.
IOLs typically have some optical power. IOLs to be placed in the posterior chamber typically have sufficient power to compensate for the removal of the natural lens. Lenses to be placed in the anterior chamber may supplement the power of the natural lens or may have power to compensate for removal of natural lens. IOLs come in a variety of configurations and materials.
Some IOLs are multifocal. Multifocal IOLs are IOLs that have at least two different optical powers. Typically, such multifocal IOLs ameliorate the effects of presbyopia by providing a wearer with optical power to permit near vision and distance vision. Multifocal IOLs where at least one of the powers is at least partially provided using a diffractive surface offer an advantage of being relatively small in profile compared to IOLs that are solely refractive. Therefore, diffractive IOLs are typically more easily injected into a patient's eye through a smaller incision.

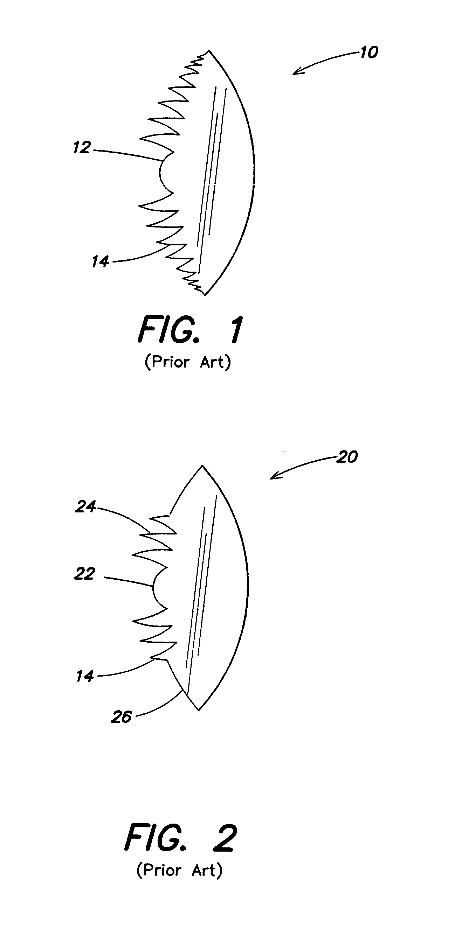

FIG. 1 illustrates a conventional multifocal IOL 10 in which a curved surface 12 has a diffractive component 14 disposed thereon that is configured such that a first portion of the light incident on the diffractive component is diffracted and focused at a first focal distance (i.e., the first portion of light is directed into the first diffractive order of the diffractive component). The first focal distance of the first portion is determined by the combined optical powers of the diffractive component and the curved (i.e., refractive) surface.
A second portion of the light passes through the diffractive component without being diffracted (i.e., the light is directed into the zeroth diffractive order of the diffractive component). The second portion of light passes through the diffractive component of the lens without deviation due to diffraction. However, the light in the zeroth order is refracted due to the curvature of surface so as to be focused at a second focal distance.
FIG. 2 illustrates a second conventional multifocal IOL 20 in which a first region 22 of one surface of the IOL has a diffractive component 24. The first region of surface is configured such that a first portion of light is projected into the first order of the diffractive component which, in combination with the refractive power of surface, provides optical power sufficient for near vision. Light passing through the first region in the zeroth order of the diffractive portion is only refracted and combines with light from the outer portion 26 of the lens for distance vision. Accordingly, light in the first diffractive order is focused at a first distance and light in the zeroth diffractive order is focused at a second distance.
In US-A-2004/0252274, a bifocal multi order diffractive lens is provided having a lens body with one or more first regions having a first multi order diffractive structure providing near vision correction, and one or more second regions having a second multi order diffractive structure providing distance vision correction.

Summary

A disadvantage of diffractive multifocal IOLs that rely on refraction of a zeroth order is that lens thickness is relatively great due to the need for a suitably curved refractive surface to focus light in the zeroth order. Another disadvantage of focusing a zeroth order of diffraction is that light that is only refracted (i.e., undiffracted light in the zeroth order) has a relatively large amount of positive chromatic aberration compared to light that is at least partially diffracted; accordingly, a positively-powered refractive surface of the IOL that passes light in the zeroth order exacerbates positive chromatic aberration provided by a patient's cornea and/o natural lens.
An IOL according to the present invention is defined by claim 1.
In some embodiments, the two or more regions cover an entire or substantially an entire lens surface. It will be appreciated that in such embodiments, none or substantially none of the light passes through the lens undiffracted. In some embodiments, diffractive surfaces may be disposed on only a portion of the anterior surface or on only a portion of the posterior surface; however, the portions are arranged such that none of the light passes through the lens undiffracted.
A first aspect of the invention is directed to an IOL, comprising a first diffractive region, and a second diffractive region having a power of a different magnitude than the first diffractive region. The first region is adapted to diffract substantially all light at a first selected wavelength projected therethrough into a single non-zero order of diffraction, and the second region is adapted to diffract substantially all light at a second selected wavelength projected therethrough

2

into a single non-zero order of diffraction.

In some embodiments, the first selected wavelength and the second selected wavelength are at substantially a same wavelength in the visible spectrum (i.e., within 100 nm of one another). In some embodiments, the first selected wavelength and the second selected wavelength are at a same wavelength in the visible spectrum (e.g., approximately 555 nm).

In some embodiments, the IOL further comprises a third diffractive region having a power of different magnitude than the first and the second regions. The third region is adapted to diffract substantially all light (at a selected wavelength) projected therethrough into a corresponding single order of diffraction.

In some embodiments, the first region and the second region project light into respective first orders of diffraction. The first and second regions may be disposed concentrically. The IOL may comprise one or more haptics. In some embodiments, the IOL is made of a material comprising at least one of silicone, PMMA, foldable hydrophilic acrylic and foldable hydrophobic acrylic. In some embodiments, the IOL further comprises a third region, concentric with the first and second regions, wherein the first region, the second region, and the third region form alternating regions of near and far focus.

A method according to the invention is defined by claim 12.

Brief Description of the Drawings

Illustrative, non-limiting embodiments of the present invention will be described by way of example with reference to the accompanying drawings, in which the same reference number is used to designate the same or similar components in different figures, and in which:

FIG. 1 is a schematic, cross-sectional illustration of a conventional multifocal diffractive IOL;
FIG. 2 illustrates another conventional multifocal, diffractive IOL;
FIG. 3A is a cross sectional view of an example of an embodiment of an IOL constructed according to aspects of the present invention; and
FIG. 3B is a plan view of the lens in FIG. 3A.

Detailed Description

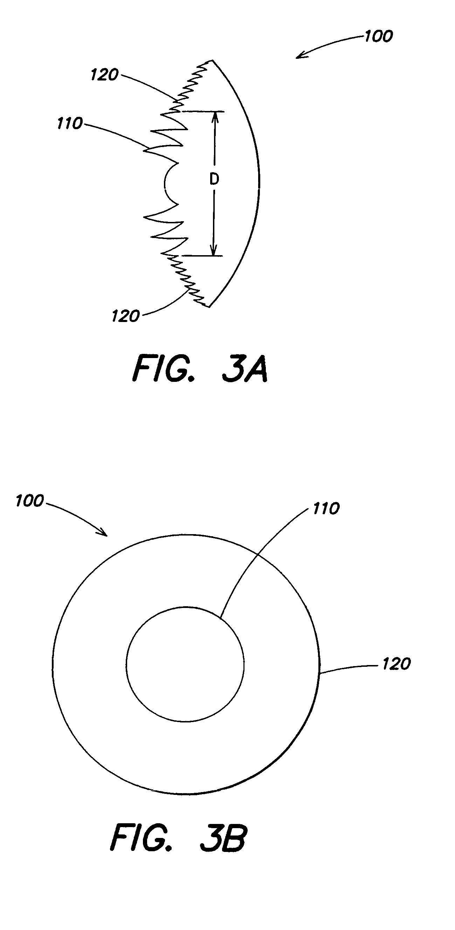

FIG. 3A is a cross sectional view of an example of an embodiment of an IOL 100 constructed according to aspects of the present invention.

IOL 100 includes a first diffractive region 110 having a diameter D, and a second diffractive region 120. First and second diffractive regions have powers of different magnitudes than one another and therefore have different focal points. The powers are selected according to conventional techniques to provide a patient vision at two different distances. For example, a first power may provide the patient with near vision for reading and a second power may be selected for distance vision. Although the illustrated lens has two regions having powers of different magnitude, any suitable number of regions may be provided (e.g., three, four or more regions). Typically when three or more regions having different powers are present, a first region provides near vision, a second provides distance vision and the remaining regions provide intermediate vision at one or more distances.

According to aspects of the invention, the first region is adapted to diffract substantially all light of a first selected wavelength projected therethrough into a single non-zero order of diffraction, and the second region is adapted to diffract substantially all light of a second selected wavelength projected therethrough into a single non-zero order of diffraction.

It will be appreciated that a lens so adapted can be constructed to be thinner than a lens adapted to project light into the zeroth order because there is no need to refract light in the zeroth order (i.e., any refractive power that may be provided is reduced due to the power provided by the diffractive surface). Typically, the non-zeroth order will be the first positive or the first negative order due to the ease of manufacture and/or efficiency with which diffraction into a first order can be achieved. However, according to aspects of the present invention, any positive or negative order of diffraction can be used to provide different powers in the regions. Diffractive regions can be formed on the anterior and/or posterior surfaces of an IOL.

In some embodiments, the first selected wavelength and the second selected wavelength are a same wavelength in the visible spectrum or substantially a same wavelength in the visible spectrum (e.g., within 20 nm of one another); however the first selected wavelength may be different than the second selected wavelength. In some embodiments in which the first selected wavelength is the same as the second selected wavelength, the wavelength is located at a wavelength of high ocular sensitivity (e.g., approximately 555 nm). Even if the first and second selected wavelength are different than one another, in some embodiments both wavelengths are located at wavelengths of high ocular sensitivity (e.g., the first selected wavelength is 555 nm and the second selected wavelength is 548 nm).

It will be appreciated that since all of the light used to form images using IOLs according to aspects of the present invention is formed at least in part by a diffractive surface, the IOL contributes a reduced amount of chromatic aberration compared to a diffractive lens that passes light through a zeroth diffraction order. In some embodiments, the lens transmits light having a reduced amount of positive chromatic aberration, and in some embodiments, the lens transmits light having negative chromatic aberration and as such is better able to compensate for positive chromatic aberration introduced by the patient's cornea, natural lens and/or other ocular media.

The term "adapted to project substantially all light at a selected wavelength projected therethrough into a single order of diffraction" means that of the light at the selected wavelength incident on a given location within a region of a lens, at least 90% of the light enters into the selected single diffraction order of that region. It will be appreciated that diffraction in a given region typically occurs with some coupling into orders other than the selected order. While a certain amount of such loss of light may be tolerated, higher transmission efficiency is typically desirable. For example, higher transmission efficiency will result in better low light vision and better contrast sensitivity. In some embodiments, at least 95% or more of the light is directed into a selected order.

Equation 1 is an equation providing a relationship between the grating element spacing and diffractive focal length for a given wavelength.

$$r_q = sqrt(2{*}q{*}\lambda{*}d) \qquad \text{Equation 1}$$

where

q = zone number

$r_q$ = radius (i.e., spacing) of a concentric annular zone

λ = design wavelength

d = focal length of diffractive element

As can be seen in the equation, the spacing of the zones, r, is proportional to the focal length, d. Since power is inversely related to focal length, zone spacing decreases to achieve greater diffractive power.

Equation 2 is an equation providing a relationship between the grating depth and the transmission efficiency for a given order of diffracted light for a given wavelength.

$$I = sinc^2(m - \alpha) \qquad \text{Equation 2}$$

where

m = diffractive order

α = (n' - n)*Δ/λ

n' = refractive index of IOL material

n = refractive index of surrounding material (i.e., aqueous humor)

Δ = maximum depth of grating

In some embodiments of the present invention, a diffractive region is designed such that peak diffraction efficiency occurs for light at a wavelength at which maximum sensitivity of an eye occurs (i.e., approximately 555 nm), and as a result, light having shorter or longer wavelengths is less efficiently diffracted. An advantage of IOL's according to such aspects is that light having wavelengths substantially shorter than 555 nm (e.g., blue light, violet light and/or ultraviolet light) is at least partially out of focus on the eye. Blue light, violet light and UV light have been associated with age-related macular degeneration. It is believed that defocused blue, violet and UV light are less damaging to a person's macula (i.e., retina), because defocused energy has reduced intensity.

It is to be appreciated that the magnitude of defocus, as a function of wavelength, that can be achieved with a diffractive region is greater than can be typically achieved due to longitudinal chromatic aberration in a refractive IOL. For example, a typical diffractive lens may provide an optical power difference of 1 diopter (positive chromatic aberration) between violet light (400 nm) and green light (555 nm), while a diffractive element may provide an optical power difference of 10 diopters (negative chromatic aberration) between violet light and green light

Furthermore, unlike blue-blocking chromophores which are commonly added to IOLs to reduce or eliminate blue light incident on a patient's retina, with IOLs according to aspects of the present invention, blue light still reaches a patient's retina. Blue light, even if it is defocused, reaching a patient's retina is believed to provide some benefits related to seasonal disorders.

FIG. 3B is a plan view of the lens 100 in FIG. 3A. It will be appreciated that the regions 110 and 120 of the lens are disposed concentrically. However, any suitable arrangement of the regions may be used. Also, although two concentrically disposed regions are shown, three, four or more concentrically disposed regions may be used in some embodiments. For example, the regions may be disposed as concentric regions, where alternate regions provide near and far focus.

Lenses according to aspects of the present invention may be configured for placement in any internal portion of a patient's eye (e.g., an anterior chamber or a posterior chamber). Although lens 100 is shown without haptics, one or more haptics may be added to facilitate positioning of an IOL in an eye. The haptics may be of any suitable shape (e.g., filament-shaped or plate-shaped). Lenses according to aspects of the invention may be made using any suitable manufacturing technique. However one suitable technique for forming the fine diffractive features is injection molding.

Lenses according to aspects of the invention may be made using any suitable material. For example, at least one of silicone, polymethyl methacrylate (PMMA), foldable hydrophilic acrylic or foldable hydrophobic acrylic acrylic may be used.

In the illustrated embodiment, light passing through all portions of the lens is diffracted. It is to be appreciated that although in the illustrated embodiment light passing through all portion of the lens will be diffracted, in any given embodiment of a lens, only portions of the lens through which light will pass, when the lens is in an eye provide advantages for chromatic aberration. Other portions of the lens (i.e., the outer periphery of the lens) may or may not be configured to diffract light.

The following is an example of a design of a 20-diopter "central add" bifocal, diffractive IOL made of silicone according to aspects of the present invention. Due to the use of diffractive power, although the lens is a 20 diopter lens, the base lens design has 15 diopters of refractive power, so it is thinner than a standard 20-diopter lens. Although the present lens is shaped to provide refractive power, in some embodiments, all optical power is provided diffractively.

The tables below show the optical prescriptions of the base lens and a diffraction grating disposed on the anterior surface of the exemple lens.

Zones 1 - 7 correspond to the central add region and have a diffractive power of 8.75 diopters and a total optical power of 23.75 diopters. Zones 8 - 40 correspond to the outer annular region and have a diffractive power of 5 diopters and a total optical power of 20 diopters. Hence the central add power is 3.75 diopters. The diameter of the central region is 1.9 mm.

Each of the first region (including zones 1-7) and the second region (including zones 8-40) are adapted to project substantially all light projected therethrough substantially into a corresponding single order of diffraction.

| | |
|---|---|
| Design Wavelength | 555 nm |
| IOL Material Refractive Index for Design Wavelength | 1.427 |
| Aqueous Humor Refractive Index for Design Wavelength | 1.336 |
| Anterior Surface Radius | 12.0996 |
| Posterior Surface Radius | -12.0996 |
| Optic Zone Diameter | 6 mm |
| Optic Body Diameter | 6 mm |
| Central Thickness | 1.056 mm |
| Edge Thickness | 0.3 mm |
| Grating Depth | 6.099 $\mu$m |

| Zone number | Radius |
|---|---|
| 1 | 0.3562 |
| 2 | 0.5037 |
| 3 | 0.6169 |
| 4 | 0.7123 |
| 5 | 0.7964 |
| 6 | 0.8724 |
| 7 | 0.9423 |
| 5 | 1.0536 |

(continued)

| Zone number | Radius |
| --- | --- |
| 6 | 1.1541 |
| 7 | 1.2466 |
| 8 | 1.3327 |
| 9 | 1.4135 |
| 10 | 1.4900 |
| 11 | 1.5627 |
| 12 | 1.6322 |
| 13 | 1.6988 |
| 14 | 1.7630 |
| 15 | 1.8248 |
| 16 | 1.8847 |
| 17 | 1.9427 |
| 18 | 1.9990 |
| 19 | 2.0538 |
| 20 | 2.1071 |
| 21 | 2.1592 |
| 22 | 2.2100 |
| 23 | 2.2596 |
| 24 | 2.3082 |
| 25 | 2.3558 |
| 26 | 2.4025 |
| 27 | 2.4483 |
| 28 | 2.4932 |
| 29 | 2.5373 |
| 30 | 2.5807 |
| 31 | 2.6234 |
| 32 | 2.6653 |
| 33 | 2.7067 |
| 34 | 2.7474 |
| 35 | 2.7875 |
| 36 | 2.8270 |
| 37 | 2.8660 |
| 38 | 2.9045 |
| 39 | 2.9424 |
| 40 | 2.9799 |

Having thus described the inventive concepts and a number of exemplary embodiments, it will be apparent to those skilled in the art that the invention may be implemented in various ways, and that modifications and improvements will readily occur to such persons. Thus, the embodiments are not intended to be limiting and presented by way of example only. The invention is limited only as required by the following claims and equivalents thereto.

**Claims**

1. An IOL comprising:

   a first diffractive region (110) having a first power; and
   a second diffractive region (120) having a second power, the first power and the second power being different than one another such that the lens being adapted to provide a patient with vision at different distances, **characterized in that**
   the first region (110) is adapted to diffract substantially all light at a first selected wavelength projected therethrough into a single non-zero order of diffraction,
   and the second region adapted to diffract substantially all light at a second selected wavelength projected therethrough into a single non-zero order of diffraction.

2. The IOL of claim 1, further comprising a third diffractive region having a power of different magnitude than the first and the second regions (110,120), adapted to diffract substantially all light at a third selected wavelength projected therethrough into a corresponding single non-zero order of diffraction.

3. The IOL of claim 1, wherein the first region and the second region project light into corresponding first orders of diffraction.

4. The IOL of claim 1, wherein the first and second regions (110,120) are disposed concentrically.

5. The IOL of claim 1, wherein the IOL comprises one or more haptics.

6. The IOL of claim 1, wherein the IOL is made of a material comprising at least one of silicone, PMMA, foldable hydrophilic acrylic and foldable hydrophobic acrylic.

7. The IOL of claim 4, further comprising a third region that is concentric with the first and second regions, wherein the first region (110), the second region (120), and the third region form alternating regions of near and far focus.

8. The IOL of claim 1, wherein the first selected wavelength and the second selected wavelength are at substantially a same wavelength in the visible spectrum.

9. The IOL of claim 1, wherein the first selected wavelength and the second selected wavelength are at a same wavelength in the visible spectrum.

10. The IOL of claim 1, wherein the same wavelength is approximately 555 nm.

11. The IOL of claim 1, wherein the first selected wavelength is a wavelength at which maximum sensitivity occurs, and the first region is designed to have a peak diffraction efficiency at the first selected wavelength and to diffract light of longer and shorter wavelengths less efficiently.

12. A method, composing:

   projecting light of a selected wavelength onto a first diffractive region (110) and a second diffractive region (120) of an IOL (100), the first region (110) having a first diffractive optical power and the second region (120) having a second diffractive optical power the first power and the second power bieng different than one another, **characterized by**;
   diffracting substantially all light of a first selected wavelength projected onto the first region (110) into a single non-zero order of diffraction; and
   diffracting substantially all light of a second selected wavelength projected onto the second region (120) into a single non-zero order of diffraction.

13. The method of claim 12, wherein the first selected wavelength and the second selected wavelength are at substantially a same wavelength in the visible spectrum.

14. The method of claim 12, wherein the first selected wavelength and the second selected wavelength are at a same wavelength in the visible spectrum.

15. The method of claim 14, wherein the same wavelength is approximately 555 nm.

16. The method of claim 12, wherein the first selected wavelength is a wavelength at which maximum sensitivity occurs, and the first region is designed to have a peak diffraction efficiency at the first selected wavelength and to diffract light of longer and shorter wavelengths less efficiently.

**Patentansprüche**

1. IOL mit:

    einem ersten diffraktiven Bereich (110) mit einer ersten Brechkraft; und
    einem zweiten diffraktiven Bereich (120) mit einer zweiten Brechkraft, wobei sich die erste Brechkraft und die zweite Brechkraft voneinander unterscheiden, so dass die Linse geeignet ist, einen Patienten bei unterschiedlichen Entfernungen mit einem Sehvermögen zu versehen, **dadurch gekennzeichnet dass** der erste Bereich (110) angepasst ist, im Wesentlichen das gesamte Licht bei einer ersten ausgewählten, dort hindurch projizierten Wellenlänge zu einer einzigen von Null verschiedenen Beugungsordnung zu beugen, und der zweite Bereich (120) angepasst ist, im Wesentlichen das gesamte Licht bei einer zweiten ausgewählten, dort hindurch projizierten Wellenlänge zu einer einzigen von Null verschiedenen Beugungsordnung zu beugen.

2. IOL nach Anspruch 1, die ferner einen dritten diffraktiven Bereich mit einer Brechkraft mit einer anderen Größe als der erste und der zweite Bereich (110, 120) aufweist, der angepasst ist, im Wesentlichen das gesamte Licht bei einer dritten ausgewählten, dort hindurch projizierten Wellenlänge zu einer entsprechenden, einzigen, von Null verschiedenen Beugungsordnung zu beugen.

3. IOL nach Anspruch 1, wobei der erste Bereich und der zweite Bereich Licht in entsprechende erste Beugungsordnungen projizieren.

4. IOL nach Anspruch 1, wobei der erste und zweite Bereich (110, 120) konzentrisch angeordnet sind.

5. IOL nach Anspruch 1, wobei die IOL eine oder mehrere Haptiken aufweist.

6. IOL nach Anspruch 1, wobei die IOL aus einem Material hergestellt ist, das mindestens eines von Silikon, PMMA, faltbarem hydrophilem Acryl und faltbarem hydrophobem Acryl aufweist.

7. IOL nach Anspruch 4, die ferner einen dritten Bereich aufweist, der mit dem ersten und zweiten Bereich konzentrisch ist, wobei der erste Bereich (110), der zweite Bereich (120) und der dritte Bereich abwechselnde Bereiche eines nahen und fernen Brennpunkts bilden.

8. IOL nach Anspruch 1, wobei sich die erste ausgewählte Wellenlänge und die zweite ausgewählte Wellenlänge im Wesentlichen bei einer selben Wellenlänge im sichtbaren Spektrum befinden.

9. IOL nach Anspruch 1, wobei sich die erste ausgewählte Wellenlänge und die zweite ausgewählte Wellenlänge bei einer selben Wellenlänge im sichtbaren Spektrum befinden.

10. IOL nach Anspruch 1, wobei dieselbe Wellenlänge annähernd 555 nm beträgt.

11. IOL nach Anspruch 1, wobei die erste ausgewählte Wellenlänge eine Wellenlänge ist, bei der eine maximale Empfindlichkeit auftritt, und der erste Bereich so gestaltet ist, dass er bei der ersten ausgewählten Wellenlänge einen höchsten Beugungswirkungsgrad aufweist und Licht mit längeren und kürzeren Wellenlängen weniger wirksam beugt.

12. Verfahren, mit den Schritten:

    Projizieren von. Licht einer ausgewählten Wellenlänge auf einen ersten diffraktiven Bereich (110) und einen zweiten diffraktiven Bereich (120) einer IOL (100), wobei der erste Bereich (110) eine erste diffraktive optische Brechkraft aufweist und der zweite Bereich (120) eine zweite diffraktive optische Brechkraft aufweist, wobei sich die erste Brechkraft und die zweite Brechkraft voneinander unterscheiden, **gekennzeichnet durch**
Beugen im Wesentlichen des gesamten Lichts einer ersten ausgewählten Wellenlänge, die auf den ersten Bereich (110) projiziert wird, zu einer einzigen von Null verschiedenen Beugungsordnung; und
Beugen im Wesentlichen des gesamten Lichts einer zweiten ausgewählten Wellenlänge, die auf den zweiten Bereich (120) projiziert wird, zu einer einzigen von Null verschiedenen Beugungsordnung.

13. Verfahren nach Anspruch 12, wobei sich die erste ausgewählte Wellenlänge und die zweite ausgewählte Wellenlänge im Wesentlichen bei einer selben Wellenlänge im sichtbaren Spektrum befinden.

14. Verfahren nach Anspruch 12, wobei sich die erste ausgewählte Wellenlänge und die zweite ausgewählte Wellenlänge bei einer selben Wellenlänge im sichtbaren Spektrum befinden.

15. Verfahren nach Anspruch 14, wobei dieselbe Wellenlänge annähernd 555 nm beträgt.

16. Verfahren nach Anspruch 12, wobei die erste ausgewählte Wellenlänge eine Wellenlänge ist, bei der eine maximale Empfindlichkeit auftritt, und der erste Bereich so gestaltet ist, dass er bei der ersten ausgewählten Wellenlänge einen höchsten Beugungswirkungsgrad aufweist und Licht mit längeren und kürzeren Wellenlängen weniger wirksam beugt.

## Revendications

1. Lentille intraoculaire comprenant :

   une première région diffractive (110) ayant une première puissance ; et
   une deuxième région diffractive (120) ayant une deuxième puissance, la première puissance et la deuxième puissance étant différentes l'une de l'autre de sorte que la lentille est conçue pour fournir à un patient une vision à différentes distances, **caractérisée en ce que** la première région (110) est conçue pour diffracter sensiblement toute la lumière à une première longueur d'onde sélectionnée projetée à travers celle-ci dans un ordre de diffraction unique non nul, et la deuxième région (120) est conçue pour diffracter sensiblement toute la lumière à une deuxième longueur d'onde sélectionnée projetée à travers celle-ci dans un ordre de diffraction unique non nul.

2. Lentille intraoculaire selon la revendication 1, comprenant en outre une troisième région diffractive ayant une puissance d'une valeur différente de celle des première et deuxième régions (110, 120), conçue pour diffracter sensiblement toute la lumière à une troisième longueur d'onde sélectionnée projetée à travers celle-ci dans un ordre de diffraction unique non nul correspondant.

3. Lentille intraoculaire selon la revendication 1, dans laquelle la première région et la deuxième région projettent la lumière dans des premiers ordres de diffraction correspondants.

4. Lentille intraoculaire selon la revendication 1, dans laquelle les première et deuxième régions (110, 120) sont disposées concentriquement.

5. Lentille intraoculaire selon la revendication 1, dans laquelle la lentille intraoculaire comprend une ou plusieurs haptiques.

6. Lentille intraoculaire selon la revendication 1, dans laquelle la lentille intraoculaire est réalisée dans un matériau comprenant au moins un des matériaux suivants : la silicone, le PMMA, l'acrylique hydrophile pliable et l'acrylique hydrophobe pliable.

7. Lentille intraoculaire selon la revendication 4, comprenant en outre une troisième région qui est concentrique avec les première et deuxième régions, dans laquelle la première région (110), la deuxième région (120) et la troisième région forment des régions alternées de focalisation de près et de focalisation de loin.

8. Lentille intraoculaire selon la revendication 1, dans laquelle la première longueur d'onde sélectionnée et la deuxième longueur d'onde sélectionnée sont sensiblement à une même longueur d'onde dans le spectre visible.

9. Lentille intraoculaire selon la revendication 1, dans laquelle la première longueur d'onde sélectionnée et la deuxième longueur d'onde sélectionnée sont à une même longueur d'onde dans le spectre visible.

10. Lentille intraoculaire selon la revendication 1, dans laquelle la même longueur d'onde est d'environ 555 nm.

11. Lentille intraoculaire selon la revendication 1, dans laquelle la première longueur d'onde sélectionnée est une longueur d'onde à laquelle la sensibilité est maximale, et la première région est conçue pour avoir un rendement de diffraction maximal à la première longueur d'onde sélectionnée et pour diffracter moins efficacement la lumière de longueurs d'ondes plus longues ou plus courtes.

12. Procédé comprenant :

la projection de lumière d'une longueur d'onde sélectionnée sur une première région diffractive (110) et sur une deuxième région diffractive (120) d'une lentille intraoculaire (100), la première région (110) ayant une première puissance optique diffractive et la deuxième région (120) ayant une deuxième puissance optique diffractive, la première puissance et la deuxième puissance étant différentes l'une de l'autre, **caractérisé par**
la diffraction sensiblement de toute la lumière d'une première longueur d'onde sélectionnée projetée sur la première région (110) dans un ordre de diffraction unique non nul ; et
la diffraction sensiblement de toute la lumière d'une deuxième longueur d'onde sélectionnée projetée sur la deuxième région (120) dans un ordre de diffraction unique non nul.

13. Procédé selon la revendication 12, dans lequel la première longueur d'onde sélectionnée et la deuxième longueur d'onde sélectionnée sont sensiblement à une même longueur d'onde dans le spectre visible.

14. Procédé selon la revendication 12, dans lequel la première longueur d'onde sélectionnée et la deuxième longueur d'onde sélectionnée sont à une même longueur d'onde dans le spectre visible.

15. Procédé selon la revendication 14, dans lequel la même longueur d'onde est d'environ 555 nm.

16. Procédé selon la revendication 12, dans lequel la première longueur d'onde sélectionnée est une longueur d'onde à laquelle se produit une sensibilité maximale, et la première région est conçue pour avoir un rendement de diffraction maximal à la première longueur d'onde sélectionnée et pour diffracter la lumière de longueurs d'ondes plus longues ou plus courtes moins efficacement.

## FIG. 1
(Prior Art)

## FIG. 2
(Prior Art)

FIG. 3A

FIG. 3B

**EP 2 162 093 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040252274 A